# EUROPÄISCHE PATENTSCHRIFT

(11) **EP 1 172 357 B1**
(45) Veröffentlichungstag und Bekanntmachung des Hinweises auf die Patenterteilung: **26.05.2004**
(21) Anmeldenummer: 01126454.6
(22) Anmeldetag: 31.01.1996
(51) Int. Cl.: C07C 281/04, C07C 281/02

(54) **Verfahren zur Herstellung von Hydrazincarbonsäureestern und Zwischenprodukte dieses Verfahrens**
Process for the preparation of hydrazinecarboxylic esters and intermediates of this process
Procédé pour la préparation d'esters hydrazinecarboxyliques et produits intermediaires de cet procédé

(30) Priorität: 13.02.1995 DE 19504627
(43) Veröffentlichungstag der Anmeldung: 16.01.2002
(62) Teilanmeldung aus: 96101303.4
(73) Patentinhaber: Bayer CropScience AG, 40789 Monheim (DE)
(72) Erfinder: Wroblowsky, Hans-Jürgen, Dr., 40764 Langenfeld (DE)

(56) Entgegenhaltungen:
- EP-A- 0 577 394
- WO-A-94/19323
- US-A- 4 282 169
- MADDING G D ET AL: "SYNTHESIS AND X-RAY CRYSTAL STRUCTURE OF A 2,4,5-TRISUBSTITUTED 1,2,4-TRIAZOLIN-3-ONE" JOURNAL OF HETEROCYCLIC CHEMISTRY, HETEROCORPORATION. PROVO, US, Bd. 22, 1. Juli 1985 (1985-07-01), Seiten 1121-1126, XP000196036 ISSN: 0022-152X
- ROECHLING H ET AL: "HETEROCYCLEN AUS 3,3-DICHLOR-2,2-DIFLUORPROPIONIMIDSÄURE- METHYLESTER" JUSTUS LIEBIGS ANNALEN DER CHEMIE, VERLAG CHEMIE GMBH. WEINHEIM, DE, 1974, Seiten 504-522, XP000569702 ISSN: 0075-4617
- ASTORGA C ET AL: "SYNTHESIS OF HYDRAZIDES THROUGH AN ENZYMATIC HYDRAZINOLYSIS REACTION" SYNTHESIS, GEORG THIEME VERLAG. STUTTGART, DE, 1. Mai 1991 (1991-05-01), Seiten 350-352, XP000196018 ISSN: 0039-7881

## Beschreibung

Die Erfindung betrifft neue Zwischenprodukte zur Herstellung von weitgehend bekannten Triazolinonen, welche als Zwischenprodukte zur Herstellung von Herbiziden und Insektiziden verwendet werden können, sowie Verfahren zur Herstellung dieser Zwischenprodukte.

Es ist bekannt, dass man bestimmte substituierte Triazolinone, wie z.B. die Verbindung 4-Methyl-5-trifluormethyl-2,4-dihydro-3H-1,2,4-triazol-3-on erhält, wenn man entsprechende Triazolinthione, wie z.B. die Verbindung 4-Methyl-5-trifluormethyl-2,4-dihydro-3H-1,2,4-triazol-3-thion, zunächst mit einem Alkylierungsmittel, wie z.B. Methyliodid, in Gegenwart eines Säurebindemittels, wie z.B. Natriummethylat umsetzt, das hierbei gebildete Alkylthiotriazolderivat auf übliche Weise isoliert, dann mit Hydrogenperoxid in Gegenwart von Essigsäure erhitzt, nach Abkühlen neutralisiert und auf übliche Weise aufarbeitet (vgl. US-P 3780052 - Example 2).

Weiter ist bekannt, dass die oben genannte Verbindung 4-Methyl-5-trifluormethyl-2,4-dihydro-3H-1,2,4-triazol-3-on auch durch Erhitzen von 1-Trifluoracetyl-4-methylsemicarbazid auf 160°C bis 180°C, anschließende Extraktion mit Essigsäureethylester und Säulenchromatographie erhalten werden kann (vgl. US-P 3780052 - Example 3).

Bei den beiden angegebenen Synthesemethoden sind jedoch Ausbeute und Qualität der erhaltenen Produkte sehr unbefriedigend.

Weitere Verfahren zur Herstellung von Triazolinonen sind Gegenstand vorgängiger, jedoch nicht vorveröffentlichter Anmeldungen (vgl. DE-4 339 412 und DE-4 342 190).

Erfindungsgemäß wurde ein neues Verfahren zur Herstellung von Triazolinonen der allgemeinen Formel (I), 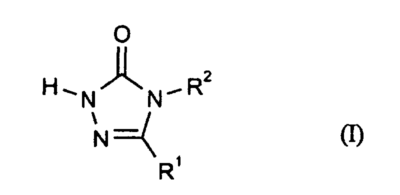 in welcher
- R¹: für Halogenalkyl mit 1 bis 6 Kohlenstoffatomen steht und
- R²: für Wasserstoff, Hydroxy oder Amino, für jeweils gegebenenfalls durch Cyano, Halogen oder C₁-C₄-Alkoxy substituiertes Alkyl, Alkenyl, Alkinyl, Alkoxy, Alkylamino oder Dialkylamino mit jeweils bis zu 6 Kohlenstoffatomen in den Alkyl-, Alkenyl- oder Alkinylgruppen, oder für jeweils gegebenenfalls durch Cyano, Halogen, C₁-C₄-Alkyl, C₁-C₄-Alkoxy oder C₁-C₄-Alkoxy-carbonyl substituiertes C₃-C₆-Cycloalkyl, C₃-C₆-Cycloalkyl-C₁-C₂-alkyl, Phenyl oder Phenyl-C₁-C₂-alkyl steht, gefunden,
welches dadurch gekennzeichnet ist, dass man Hydrazincarbonsäureester der allgemeinen Formel (II), 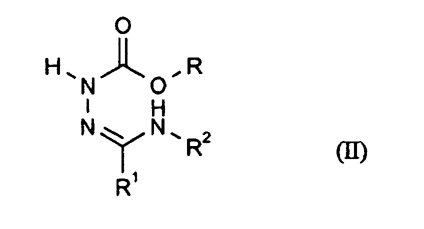 in welcher
- R¹ und R²: die oben angegebenen Bedeutungen haben und
- R: für jeweils gegebenenfalls durch Halogen oder C₁-C₄-Alkoxy substituiertes Alkyl mit 1 bis 4 Kohlenstoffatomen oder für gegebenenfalls durch Halogen oder C₁-C₄-Alkyl substituiertes Phenyl steht,
- und/oder zu den Verbindungen der Formel (II) tautomere Verbindungen -
in Gegenwart eines basischen Reaktionshilfsmittels und in Gegenwart eines Verdünnungsmittels bei Temperaturen zwischen 0°C und 150°C umsetzt.

Überraschenderweise können nach dem erfindungsgemäßen Verfahren die Triazolinone der allgemeinen Formel (I) auf einfache Weise in sehr hohen Ausbeuten erhalten werden, wobei die Substituenten breit variiert werden können.

Sicherheitstechnische Probleme, wie die Verwendung von Hydrogenperoxid in älteren Verfahren, und größere Abwasserprobleme, wie sie bei schwefelhaltigen Abwässern in älteren Verfahren auftreten, werden dabei vermieden.

Das erfindungsgemäße Verfahren stellt somit eine wertvolle Bereicherung des Standes der Technik dar.

Die bei den Restedefinitionen genannten Kohlenwasserstoffreste, wie Alkyl, auch in Kombinationen mit Heteroatomen, wie in Alkoxy, Alkylthio oder Alkylamino, sind auch dann, wenn dies nicht ausdrücklich angegeben ist, geradkettig oder verzweigt.

Halogen steht im Allgemeinen für Fluor, Chlor, Brom oder Iod, vorzugsweise für Fluor, Chlor oder Brom, insbesondere für Fluor oder Chlor.

Nach dem erfindungsgemäßen Verfahren werden insbesondere Verbindungen der Formel (I) hergestellt,
in welcher
- R¹: für jeweils durch Fluor und/oder Chlor substituiertes Methyl, Ethyl, n- oder i-Propyl, n-, i- oder s-Butyl steht und
- R²: für jeweils gegebenenfalls durch Fluor, Chlor, Cyano, Methoxy oder Ethoxy substituiertes Methyl, Ethyl, n- oder i-Propyl, n-, i- oder s-Butyl, Allyl, Propargyl, Methoxy, Ethoxy, n- oder i-Propoxy, n-, i- oder s-Butoxy, Methylamino, Ethylamino, n- oder i-Propylamino, n-, i- oder s-Butylamino, Dimethylamino oder Diethylamino oder für jeweils gegebenenfalls durch Fluor, Chlor, Brom, Cyano, Methyl, Ethyl, n- oder i-Propyl, Methoxycarbonyl oder Ethoxycarbonyl substituiertes Cyclopropyl, Cyclobutyl, Cyclopentyl, Cyclohexyl, Phenyl oder Benzyl steht.

Eine ganz besonders bevorzugte Gruppe von Verbindungen, welche nach dem erfindungsgemäßen Verfahren hergestellt werden können, sind die Verbindungen der Formel (I),
in welcher
- R¹: für jeweils entsprechend einfach, zweifach, dreifach, vierfach oder fünffach durch Fluor und/oder Chlor substituiertes Methyl oder Ethyl steht und
- R²: für Methyl, Ethyl, n- oder i-Propyl, Methoxy, Ethoxy, Methylamino, Ethylamino, Dimethylamino oder Cyclopropyl steht.

Die oben aufgeführten allgemeinen oder in Vorzugsbereichen angegebenen Restedefinitionen gelten sowohl für die Endprodukte der Formel (I) als auch entsprechend für die jeweils zur Herstellung benötigten Ausgangsstoffe bzw. Zwischenprodukte. Diese Restedefinitionen können untereinander, also auch zwischen den angegebenen Bereichen bevorzugter Verbindungen, beliebig kombiniert werden.

Verwendet man beispielsweise 2-(2,2-Difluor-1-ethylimino-ethyl)-hydrazin-1-carbonsäure-ethylester als Ausgangsstoff, so kann der Reaktionsablauf beim erfindungsgemäßen Verfahren durch das folgende Formelschema skizziert werden:

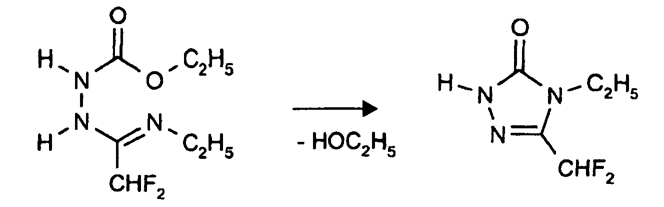

Die beim erfindungsgemäßen Verfahren zur Herstellung der Verbindungen der allgemeinen Formel (I) als Ausgangsstoffe zu verwendenden Hydrazincarbonsäureester sind durch die Formel (II) allgemein definiert. In der Formel (II) haben R¹ und R² vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für R¹ und R² angegeben wurden;

R steht vorzugsweise für gegebenenfalls durch Halogen oder C₁-C₄-Alkoxy substituiertes Alkyl mit 1 bis 4 Kohlenstoffatomen oder für gegebenenfalls durch Halogen oder C₁-C₄-Alkyl substituiertes Phenyl, insbesondere für Methyl, Ethyl, Methoxyethyl, Ethoxyethyl oder Phenyl.

Man erhält die Verbindungen der allgemeinen Formel (II), wenn man entsprechende Hydrazincarbonsäureester der allgemeinen Formel (III), 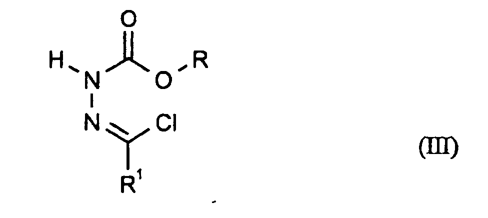 in welcher
- R und R¹: die oben angegebenen Bedeutungen haben,
mit Aminoverbindungen der allgemeinen Formel (IV),

H₂N-R² (IV)

in welcher
- R²: die oben angegebene Bedeutung hat,
gegebenenfalls in Gegenwart eines Säureakzeptors, wie z.B. Triethylamin und gegebenenfalls in Gegenwart eines Verdünnungsmittels, wie z.B. Acetonitril oder Dioxan, bei Temperaturen zwischen 0°C und 100°C umsetzt (vgl. die Herstellungsbeispiele). Dieses neue Verfahren ist Gegenstand der vorliegenden Anmeldung.

Die als Vorprodukte zu verwendenden Hydrazincarbonsäureester sind durch die Formel (III) allgemein definiert. In der Formel (III) haben R¹ und R insbesondere diejenigen Bedeutungen, die bereits oben im Zusammenhang mit der Beschreibung der Verbindungen der Formel (I) bzw. der Formel (II) als insbesondere bevorzugt für R¹ und R angegeben wurden.

Die Hydrazincarbonsäureester der Formel (III) sind noch nicht aus der Literatur bekannt; sie sind als neue Stoffe Gegenstand der vorliegenden Anmeldung.

Man erhält die neuen Verbindungen der allgemeinen Formel (III), wenn man entsprechende Hydrazincarbonsäureester der allgemeinen Formel (V), 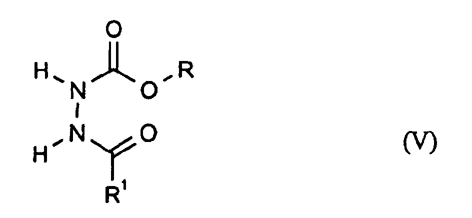 in welcher
- R und R¹: die oben angegebenen Bedeutungen haben,
mit einem Sulfonsäurechlorid, wie z.B. Methan-, Ethan-, Benzol- oder p-Toluol-sulfonsäurechlorid, gegebenenfalls in Gegenwart eines Säureakzeptors, wie z.B. Triethylamin oder N,N-Dimethyl-benzylamin, und gegebenenfalls in Gegenwart eines Verdünnungsmittels, wie z.B. Dimethoxyethan, Dioxan, Tetrahydrofuran, Methylenchlorid, Essigsäureethylester, Toluol, Chlorbenzol, Aceton, Methylethylketon, Methyl-isopropylketon, Methyl-isobutylketon, Acetonitril, Propionitril oder Butyronitril, bei Temperaturen zwischen 0°C und 100°C umsetzt (vgl. die Herstellungsbeispiele).

Die hierbei als Vorprodukte zu verwendenden Hydrazincarbonsäureester sind durch die Formel (V) allgemein definiert. In der Formel (V) haben R¹ und R vorzugsweise bzw. insbesondere diejenigen Bedeutungen, die bereits oben im Zusammenhang mit der Beschreibung der Verbindungen der Formel (I) bzw. der Formel (II) als bevorzugt bzw. als insbesondere bevorzugt für R¹ und R angegeben wurden.

Die Hydrazincarbonsäureester der Formel (V) sind bekannt und/oder können nach bekannten Verfahren hergestellt werden (vgl. Synthesis 1991, 350-352; FR- 2 123 205).

Noch nicht aus der Literatur bekannt sind die Hydrazincarbonsäureester der Formel (Va), 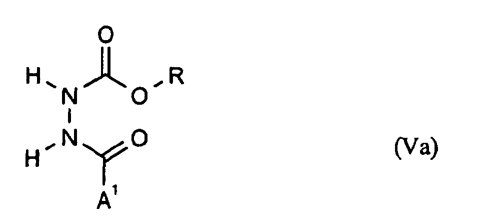 in welcher
- A¹: für Difluormethyl, Trifluormethyl, Difluormethyl oder Trifluormethyl steht und
- R: die oben angegebene Bedeutung hat.

Man erhält die neuen Hydrazincarbonsäureester der Formel (Va), wenn man Hydrazincarbonsäureester der Formel (VI), 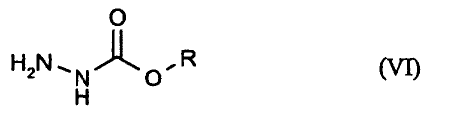 in welcher
- R: die oben angegebene Bedeutung hat,
mit Carbonsäuren oder deren Derivaten der allgemeinen Formel (VII),

A¹-CO-X (VII)

in welcher
- A¹: die oben angegebene Bedeutung hat und
- X: für Hydroxy, Halogen (insbesondere Fluor oder Chlor) oder die Gruppierung -O-CO-A¹ steht,
gegebenenfalls in Gegenwart eines Verdünnungsmittels, wie z.B. Diethylether, Acetonitril, Propionitril, Butyronitril, Chlorbenzol, Xylol oder Toluol, bei Temperaturen zwischen 0°C und 150°C umsetzt (vgl. die Herstellungsbeispiele).

Die Vorprodukte der Formeln (VI) und (VII) sind bekannte Synthesechemikalien.

Das erfindungsgemäße Verfahren wird in Gegenwart eines basischen Reaktionshilfsmittels durchgeführt. Als solche kommen alle üblichen anorganischen oder organischen Basen in Frage. Hierzu gehören beispielsweise Alkalimetall- oder Erdalkalimetall- hydride, -hydroxide, -amide, -alkoholate, -acetate, -carbonate oder -hydrogencarbonate, wie beispielsweise Lithium-, Natrium-, Kalium- oder Calcium-hydrid, Lithium-, Natrium-, Kalium- oder Calcium-hydroxid, Lithium-, Natrium- oder Kalium-amid, Natrium- oder Kalium-methylat, Natrium- oder Kalium-ethylat, Natrium- oder Kaliumpropylat, Aluminiumisopropylat, Natrium- oder Kalium-tert-butylat, Natrium- oder Kalium-hydroxid, Ammoniumhydroxid, Natrium-, Kalium- oder Calcium-acetat, Ammoniumacetat, Natrium-, Kalium- oder Calcium-carbonat, Ammoniumcarbonat, Natrium- oder Kalium-hydrogencarbonat, sowie basische organische Stickstoffverbindungen, wie Trimethylamin, Triethylamin, Tripropylamin, Tributylamin, Ethyl-diisopropylamin, N,N-Dimethylcyclohexylamin, Dicyclohexylamin, Ethyl-dicyclohexylamin, N,N-Dimethylanilin, N,N-Dimethyl-benzylamin, Pyridin, 2-Methyl-, 3-Methylund 4-Methyl-pyridin, 2,4-Dimethyl-, 2,6-Dimethyl-, 3,4-Dimethyl- und 3,5-Dimethylpyridin, 5-Ethyl-2-methyl-pyridin, N-Methyl-piperidin, 4-Dimethylamino-pyridin, Diazabicyclooctan (DABCO), Diazabicyclononen (DBN) oder Diazabicycloundecen (DBU).

Alkalimetallhydroxide, wie Natrium- oder Kaliumhydroxid oder Alkalimetallalkoholate, wie Natriummethylat, werden als basische Reaktionshilfsmittel beim erfindungsgemäßen Verfahren besonders bevorzugt.

An Stelle der oben angegebenen basischen Reaktionshilfsmittel können auch die zur Herstellung der Ausgangsstoffe der Formel (II) einzusetzenden Aminoverbindungen der Formel (IV) - oben - als basische Reaktionshilfsmittel verwendet werden.

Als Verdünnungsmittel zur Durchführung des erfindungsgemäßen Verfahrens kommen neben Wasser die üblichen organischen Lösungsmittel in Betracht. Hierzu gehören insbesondere aliphatische, alicyclische oder aromatische, gegebenenfalls halogenierte Kohlenwasserstoffe, wie beispielsweise Pentan, Hexan, Heptan, Petrolether, Ligroin, Benzin, Benzol, Toluol, Xylol, Chlorbenzol, Dichlorbenzol, Cyclohexan, Methylcyclohexan, Dichlormethan, Chloroform, Tetrachlormethan; Ether, wie Diethylether, Diisopropylether, t-Butyl-methylether, t-Pentyl-methylether, Dioxan, Tetrahydrofuran, Ethylenglykol-dimethyl- oder -diethylether, Diethylenglykol-dimethylether oder -diethylether; Ketone, wie Aceton, Butanon oder Methyl-isobutyl-keton; Nitrile, wie Acetonitril, Propionitril, Butyronitril oder Benzonitril; Amide, wie N,N-Dimethylformamid, N,N-Dimethylacetamid, N-Methylformanilid, N-Methyl-pyrrolidon oder Hexamethylphosphorsäuretriamid; Ester wie Essigsäure-methylester, -ethylester, -n- oder -ipropylester, -n-, -i- oder -s-butylester, Sulfoxide, wie Dimethylsulfoxid; Alkohole, wie Methanol, Ethanol, n- oder i-Propanol, n-, i-, s- oder t-Butanol, Ethylenglykolmonomethylether oder -monoethylether, Diethylenglykolmonomethylether, Diethylenglykolmonoethylether; deren Gemische mit Wasser oder reines Wasser.

Wasser und Methanol werden als Verdünnungsmittel beim erfindungsgemäßen Verfahren besonders bevorzugt.

Die Reaktionstemperaturen können bei der Durchführung des erfindungsgemäßen Verfahrens in einem größeren Bereich variiert werden. Im Allgemeinen arbeitet man bei Temperaturen zwischen 0°C und 150°C, vorzugsweise bei Temperaturen zwischen 10°C und 120°C, insbesondere bei Temperaturen zwischen 20°C und 110°C.

Das erfindungsgemäße Verfahren wird im Allgemeinen unter Normaldruck durchgeführt. Es ist jedoch auch möglich, unter erhöhtem oder vermindertem Druck - im Allgemeinen zwischen 0, 1 bar und 10 bar - zu arbeiten.

Zur Durchführung des erfindungsgemäßen Verfahrens zur Herstellung der Verbindungen der Formel (I) setzt man pro mol Hydrazincarbonsäureester der Formel (II) im Allgemeinen 1 bis 3 mol, vorzugsweise 1,5 bis 2,5 mol basisches Reaktionshilfsmittel ein.

In einer bevorzugten Ausführungsform des erfindunggemäßen Verfahrens wird der Hydrazincarbonsäureester der Formel (II) bei Raumtemperatur (ca. 20°C) in einem geeigneten Verdünnungsmittel aufgenommen und nach Zugabe eines basischen Reaktionshilfsmittels bei erhöhter Temperatur bis zum Ende der Umsetzung gerührt. Dann wird wieder auf Raumtemperatur (ca. 20°C) abgekühlt und mit einer starken Säure, wie z.B. Salzsäure oder Schwefelsäure angesäuert. Falls das Produkt der Formel (I) hierbei kristallin anfällt, kann es durch Absaugen isoliert werden. Andernfalls wird das Produkt der Formel (I) mit einem mit Wasser praktisch nicht mischbaren organischen Lösungsmittel, wie z.B. Essigsäureethylester extrahiert und von der organischen Phase wird dann - gegebenenfalls nach Trocknen und Filtrieren - das Lösungsmittel unter vermindertem Druck abdestilliert, wobei dann das Produkt der Formel (I) im Rückstand verbleibt. In einer weiteren bevorzugten Ausführungsform des erfindungsgemäßen Verfahrens werden - sozusagen in einer "Ein-Topf-Variante" - die als Ausgangsstoffe benötigten Hydrazincarbonsäureester der Formel (II) durch Umsetzung von Hydrazincarbonsäureestem der Formel (III) mit Aminoverbindungen der Formel (IV) wie oben beschrieben hergestellt und anschließend ohne Zwischenisolierung - "in situ" - in die Triazolinone der Formel (I) umgewandelt.

Hierzu wird vorzugsweise zunächst ein Hydrazincarbonsäureester der Formel (III) bei mäßig erhöhter Temperatur - vorzugsweise zwischen 25°C und 35°C - zu einer Mischung aus überschüssiger Aminoverbindung der Formel (IV) - im Allgemeinen zwischen 2 und 10 mol, vorzugsweise zwischen 2,5 und 5,0 mol - und einem geeigneten Verdünnungsmittel - vorzugsweise Wasser - gegeben. Dann wird das Reaktionsgemisch bei weiter erhöhter Temperatur bis zum Ende der Umsetzung erhitzt und wie oben beschrieben aufgearbeitet.

Die nach dem erfindungsgemäßen Verfahren herzustellenden Triazolinone der Formel (I) können als Zwischenprodukte zur Herstellung von landwirtschaftlich nutzbaren Wirkstoffen verwendet werden (vgl. US-P-3 780 052, US-P-3 780 053, US-P-3 780 054 und EP-A-341489).

### Herstellungsbeispiele:

### Beispiel 1

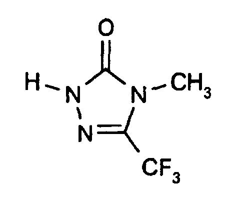

36 g (185 mmol) 2-(2,2,2-Trifluor-1-methylimino-ethyl)-hydrazin-1-carbonsäure-methylester werden in 200 ml Wasser suspendiert und nach Zugabe von 37,5 g 45 %iger wässriger Natronlauge (entsprechend 370 mmol NaOH) wird die Mischung 3 Stunden unter Rückfluss erhitzt. Nach Abkühlen auf Raumtemperatur (ca. 20°C) wird mit konz. Salzsäure angesäuert und dreimal mit je 100 ml Essigsäureethylester extrahiert. Die vereinigten organischen Extraktionslösungen werden mit Natriumsulfat getrocknet und filtriert. Vom Filtrat wird das Lösungsmittel im Wasserstrahlvakuum sorgfältig abdestilliert.

Man erhält 28,9 g (93,5 % der Theorie) 4-Methyl-5-trifluormethyl-2,4-dihydro-3H-1,2,4-triazol-3-on als festen Rückstand.

¹H-NMR (DMSO-D₆, δ): 3,256 ppm (s, 3H), 12,655 ppm (s, 1H).

### Beispiel 2

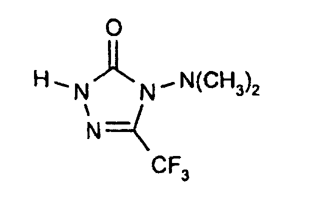

25,4 g (110 mmol) 2-(2,2,2-Trifluor-1-dimethylamino-ethyliden)-hydrazin-1-carbonsäure-methylester werden in 200 ml Wasser suspendiert und nach Zugabe von 19,8 g 45 %iger wässriger Natronlauge (entsprechend 223 mmol NaOH) wird die Mischung 3 Stunden unter Rückfluss erhitzt. Nach Abkühlen auf Raumtemperatur (ca. 20°C) wird mit konz. Salzsäure angesäuert und das kristallin angefallene Produkt durch Absaugen isoliert.

Man erhält 19,1 g (89 % der Theorie) 4-Dimethylamino-5-trifluormethyl-2,4-dihydro-3H-1,2,4-triazol-3-on vom Schmelzpunkt 138°C.

### Beispiel 3

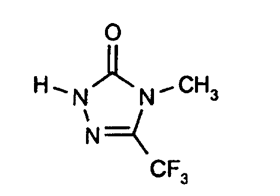

10,2 g (50 mmol) 2-(1-Chlor-2,2,2-trifluor-ethyliden)-hydrazin-1-carbonsäure-methylester werden portionsweise zu einer Mischung aus 50 ml Wasser und 22,5 g 34 %iger wässriger Methylamin-Lösung (entsprechend 250 mmol Methylamin) gegeben, wobei die Temperatur der Mischung zwischen 25°C und 35°C gehalten wird. Anschließend wird die Reaktionsmischung 2 Stunden unter Rückfluss erhitzt und dann - nach Abkühlen auf Raumtemperatur (ca. 20°C) mit konz. Salzsäure angesäuert. Dann wird dreimal mit Essigsäureethylester extrahiert. Die vereinigten Extraktionslösungen werden mit Natriumsulfat getrocknet und filtriert. Vom Filtrat wird dann das Lösungsmittel im Wasserstrahlvakuum sorgfältig abdestilliert.

Man erhält 7,3 g (85 % der Theorie) 4-Methyl-5-trifluormethyl-2,4-dihydro-3H-1,2,4-triazol-3-on als festen Rückstand.
(Gehalt: 97,0 %).

Analog zu den Beispielen 1 bis 3 sowie entsprechend der allgemeinen Beschreibung des erfindungsgemäßen Herstellungsverfahrens können beispielsweise auch die in der nachstehenden Tabelle 1 aufgeführten Verbindungen der Formel (I) hergestellt werden.

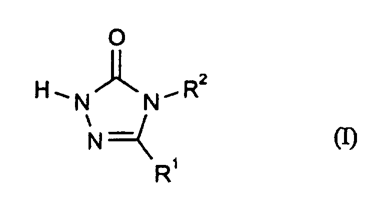

**Tabelle 1:**

| Beispiele für die erfindungsgemäß erhaltenen Verbindungen der Formel (I) | | | | |
|---|---|---|---|---|
| **Beisp.- Nr.** | **R**^{**1**} | **R**^{**2**} | **Ausbeute (% der Theorie)** | **Physikal. Daten** |
| 4 | CF₃ | (CH₂)₂OCH₃ | 92 | n_{D}²⁰=1,4290 |
| 5 | CF₃ | (CH₂)₃OCH₃ | 90 | n_{D}²⁰=1,4320 |
| 6 | CHF₂ | CH₃ | | |
| 7 | CF₂Cl | CH₃ | | |
| 8 | CF₃ | H | 70 | Fp.: 198°C |

### Ausgangsstoffe der Formel (II)

### Beispiel (II-1)

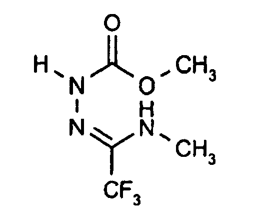

39,6 g (185 mmol) 2-(1-Chlor-2,2,2-trifluor-ethyliden)-hydrazin-1-carbonsäure-methylester werden in 220 ml Dioxan gelöst und zu dieser Lösung werden bei 20°C 50 g einer 34,5 %igen wässrigen Lösung von Methylamin (entsprechend 556 mmol Methylamin) innerhalb von 30 Minuten tropfenweise gegeben, wobei die Innentemperatur zwischen 25°C und 35°C gehalten wird. Die Mischung wird dann noch 2 Stunden bei ca. 30°C gerührt und anschließend im Wasserstrahlvakuum eingeengt. Der Rückstand wird mit 150 ml Wasser verrührt und das kristallin angefallene Produkt durch Absaugen isoliert.

Man erhält 36 g (98 % der Theorie) 2-(2,2,2-Trifluor-1-methylimino-ethyl)-hydrazin-1-carbonsäure-methylester.
¹H-NMR (DMSO-D₆, δ): 2,817 ppm (m, 3H), 3,641 ppm (s, 3H), 6,483 ppm (br. s, 1H), 9,527 ppm (s, 1H).

### Beispiel (II-2)

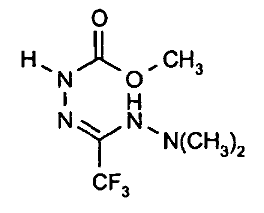

8,06 g (134 mmol) N,N-Dimethyl-hydrazin und 17 ml (122 mmol) Triethylamin werden in 120 ml Acetonitril vorgelegt und bei einer Innentemperatur von ca. 55°C wird eine Lösung von 25 g (122 mmol) 2-(1-Chlor-2,2,2-trifluor-ethyliden)-hydrazin-1-carbonsäure-methylester in 30 ml Acetonitril unter Rühren tropfenweise dazu gegeben. Die Mischung wird dann noch eine Stunde bei 60°C gerührt und anschließend im Wasserstrahlvakuum eingeengt. Der Rückstand wird in Essigsäureethylester aufgenommen, mit Wasser gewaschen, mit Natriumsulfat getrocknet und filtriert. Das Filtrat wird im Wasserstrahlvakuum eingeengt, der Rückstand durch Digerieren mit Diisopropylether zur Kristallisation gebracht und das kristalline Produkt durch Absaugen isoliert.

Man erhält 16,1 g (58 % der Theorie) 2-(2,2,2-Trifluor-1-(N,N-dimethylhydrazino)-ethyl)-hydrazin-1-carbonsäure-methylester vom Schmelzpunkt 124°C.

Analog zu den Beispielen (II-1) und (II-2) können beispielsweise auch die in der nachstehenden Tabelle 2 aufgeführten Verbindungen der Formel (II) hergestellt werden.

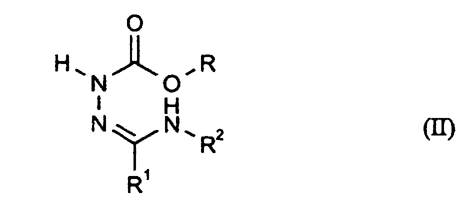

**Tabelle 2:**

| Beispiele für die Verbindungen der Formel (II) | | | | | |
|---|---|---|---|---|---|
| **Beisp.-Nr**. | **R**^{**1**} | **R**^{**2**} | **R** | **Ausbeute (% derTheorie)** | **Schmelzpunkt (°C)** |
| II-3 | CF₃ | CH₃ | CH₃ | 88 | 213 |
| II-4 | CF₃ | (CH₂)₂OCH₃ | CH₃ | 68 | 150 |
| II-5 | CF₃ | (CH₂)₃OCH₃ | CH₃ | 84 | 148 |
| II-6 | CF₃ | OH | CH₃ | 63 | 183 |
| II-7 | CF₃ | OCH₃ | CH₃ | 27 | 104 |
| II-8 | CHF₂ | CH₃ | CH₃ | | |
| II-9 | CF₂Cl | CH₃ | CH₃ | | |

### Vorprodukte der Formel (III):

### Beispiel (III-1)

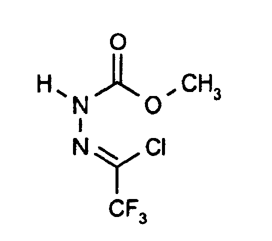

65,1 g (350 mmol) 2-Trifluoracetyl-hydrazin-1-carbonsäure-methylester und 66,2 g (375 mmol) Benzolsulfonsäurechlorid werden in 450 ml Aceton vorgelegt und bei einer Innentemperatur zwischen 50°C und 55°C wird unter Rühren eine Lösung von 48,7 ml (350 mmol) Triethylamin in 50 ml Aceton zugetropft. Nach einstündigem Rühren bei 50°C bis 55°C werden 2,5 ml Triethylamin zur Reaktionsmischung gegeben und das Rühren wird weitere 30 Minuten fortgesetzt. Nach Abkühlen mit einem Eisbad wird das kristallin angefallene Triethylammoniumchlorid durch Absaugen abgetrennt. Das Filtrat wird im Wasserstrahlvakuum eingeengt, der Rückstand in 300 ml Essigsäureethylester aufgenommen, diese Lösung dreimal mit je 100 ml Wasser gewaschen, mit Natriumsulfat getrocknet und filtriert. Vom Filtrat wird das Lösungsmittel im Wasserstrahlvakuum sorgfältig abdestilliert.

Man erhält 72,5 g (Gehalt: 92,2 %, Ausbeute: 93 % der Theorie) 2-(1-Chlor-2,2,2-trifluor-ethyliden)-hydrazin-1-carbonsäure-methylester als festes Produkt.
¹H-NMR (DMSO-D₆, δ): 3,78 ppm (s, 3H), 11,59 ppm (s, 1H).

Analog Beispiel (III-1) können beispielsweise auch die in der nachstehenden Tabelle 3 aufgeführten Verbindungen der Formel (III) hergestellt werden.

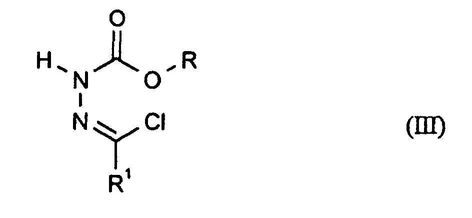

**Tabelle 3:**

| Beispiele für die Verbindungen der Formel (III) | | | | |
|---|---|---|---|---|
| **Bsp-Nr.** | **R**^{**1**} | **R** | **Ausbeute (% der Theorie)** | **Schmelzpunkt (°C)** |
| III-2 | CHF₂ | CH₃ | | |
| III-3 | CF₂Cl | CH₃ | | |

### Vorprodukte der Formel (V):

### Beispiel (V-1)

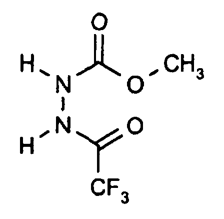

121,5 g (1,28 mol) Carbazinsäure-methylester (Hydrazincarbonsäure-methylester) werden in 1200 ml Diethylether vorgelegt und bei 0°C werden 310 g (1,48 mol) Trifluoressigsäureanhydrid innerhalb von 2 Stunden tropfenweise dazu gegeben. Dann wird die Mischung noch ca. 90 Minuten bei 0°C bis 20°C gerührt, anschließend im Wasserstrahlvakuum eingeengt, der Rückstand mit 500 ml Toluol verrührt und das kristalline Produkt durch Absaugen isoliert.

Man erhält 219 g (Gehalt: 94,4%, Ausbeute: 94 % der Theorie) 2-Trifluoracetylhydrazin-1-carbonsäure-methylester vom Schmelzpunkt 105°C.
¹H-NMR (DMSO-D₆, δ): 3,647 ppm (s, 3H), 9,649 ppm (s, 1H), 11,429 ppm (s, 1H).

### Beispiel (V-2)

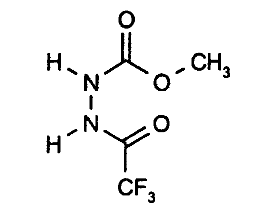

18,5 g (0,20 mol) Carbazinsäure-methylester (Hydrazincarbonsäure-methylester) werden in 150 ml Toluol vorgelegt und bei einer Innentemperatur von 80°C werden 23,1 ml (0,30 mol) Trifluoressigsäure innerhalb von 35 Minuten tropfenweise dazu gegeben. Die Mischung wird dann weitere 30 Minuten bei 80°C gerührt, anschließend eine Stunde am Wasserabscheider erhitzt und dann im Wasserstrahlvakuum eingeengt. Der Rückstand mit 500 ml Toluol verrührt und das kristalline Produkt durch Absaugen isoliert.

Man erhält 36,4 g (Gehalt: 90,1 %, Ausbeute: 88,2 % der Theorie) 2-Trifluoracetylhydrazin-1-carbonsäure-methylester vom Schmelzpunkt 105°C.

## Patentansprüche

1. Verfahren zur Herstellung von Hydrazincarbonsäureestern der allgemeinen Formel (II) 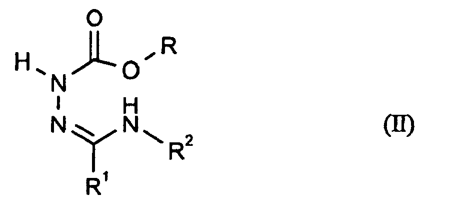 in welcher
R für gegebenenfalls durch Halogen oder C₁-C₄-Alkoxy substituiertes Alkyl mit 1 bis 4 Kohlenstoffatomen oder für gegebenenfalls durch Halogen oder C₁-C₄-Alkyl substituiertes Phenyl steht,
R¹ für Halogenalkyl mit 1 bis 6 Kohlenstoffatomen steht und
R² für Wasserstoff, Hydroxy oder Amino, für jeweils gegebenenfalls durch Cyano, Halogen oder C₁-C₄-Alkoxy substituiertes Alkyl, Alkenyl, Alkinyl, Alkoxy, Alkylamino oder Dialkylamino mit jeweils bis zu 6 Kohlenstoffatomen in den Alkyl-, Alkenyl- oder Alkinylgruppen, oder für jeweils gegebenenfalls durch Cyano, Halogen, C₁-C₄-Alkyl, C₁-C₄-Alkoxy oder C₁-C₄-Alkoxy-carbonyl substituiertes C₃-C₆-Cycloalkyl, C₃-C₆-Cycloalkyl-C₁-C₂-alkyl, Phenyl oder Phenyl-C₁-C₂-alkyl steht,
**dadurch gekennzeichnet, dass** man entsprechende Hydrazincarbonsäureester der allgemeinen Formel (III), 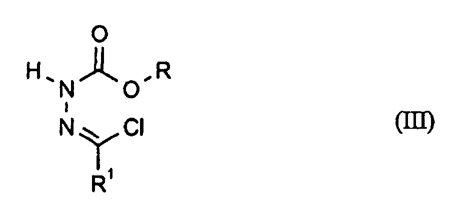 in welcher
R und R¹ die oben angegebenen Bedeutungen haben,
mit Aminoverbindungen der allgemeinen Formel (IV),
H₂N-R² (IV)
in welcher
R² die oben angegebene Bedeutung hat,
gegebenenfalls in Gegenwart eines Säureakzeptors und gegebenenfalls in Gegenwart eines Verdünnungsmittels bei Temperaturen zwischen 0°C und 100°C umsetzt.

2. Hydrazincarbonsäureester der allgemeinen Formel (III), 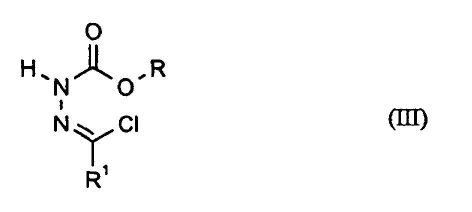 in welcher
R und R¹ wie in Anspruch 1 definiert sind.

3. Verfahren zur Herstellung von Hydrazincarbonsäureestem der allgemeinen Formel (III), 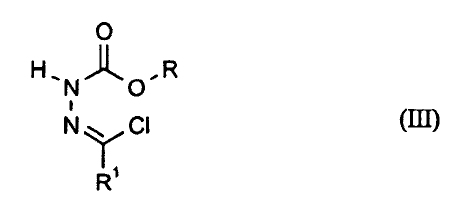 in welcher
R und R¹ die in Anspruch 2 genannten Bedeutungen haben,
**dadurch gekennzeichnet, dass** man entsprechende Hydrazincarbonsäureester der allgemeinen Formel (V), 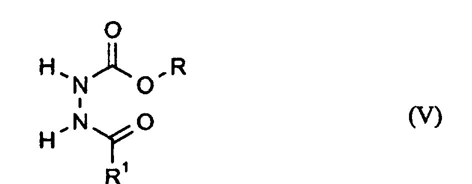 in welcher
R und R¹ die oben angegebenen Bedeutungen haben,
mit einem Sulfonsäurechlorid gegebenenfalls in Gegenwart eines Säureakzeptors und gegebenenfalls in Gegenwart eines Verdünnungsmittels bei Temperaturen zwischen 0°C und 100°C umsetzt.

## Claims

1. Process for preparing hydrazinecarboxylic esters of the general formula (II) 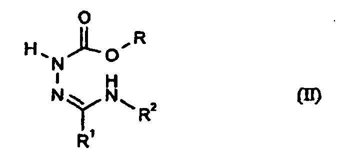 in which
R represents alkyl having 1 to 4 carbon atoms which is optionally substituted by halogen or C₁-C₄-alkoxy, or represents phenyl which is optionally substituted by halogen or C₁-C₄-alkyl,
R¹ represents halogenoalkyl having from 1 to 6 carbon atoms, and
R2 represents hydrogen, hydroxyl or amino, represents alkyl, alkenyl, alkynyl, alkoxy, alkylamino or dialkylamino which have in each case up to 6 carbon atoms in the alkyl, alkenyl or alkynyl groups and are in each case optionally substituted by cyano, halogen or C₁-C₄-alkoxy, or represents C₃-C₆-cycloalkyl, C₃-C₆-cycloalkyl-C₁-C₂-alkyl, phenyl or phenyl-C₁-C₂-alkyl which are in each case optionally substituted by cyano, halogen, C₁-C₄-alkyl, C₁-C₄-alkoxy or C₁-C₄-alkoxycarbonyl,
**characterized in that**
corresponding hydrazinecarboxylic esters of the general formula (III) 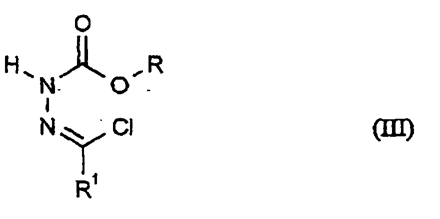 in which
R and R¹ have the abovementioned meanings,
are reacted with amino compounds of the general formula (IV)
H₂N-R² (IV)
in which
R² has the abovementioned meaning,
where appropriate in the presence of an acid acceptor and where appropriate in the presence of a diluent, at temperatures of between 0°C and 100°C.

2. Hydrazinecarboxylic esters of the general formula (III) 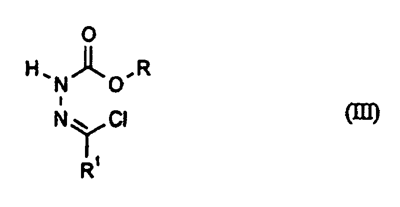 in which
R and R¹ are as defined in Claim 1.

3. Process for preparing hydrazinecarboxylic esters of the general formula (III) 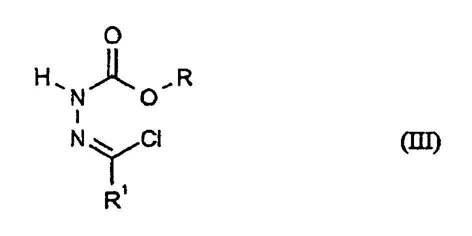 in which
R and R¹ have the meanings given in Claim 2,
**characterized in that** corresponding hydrazinecarboxylic esters of the general formula (V) 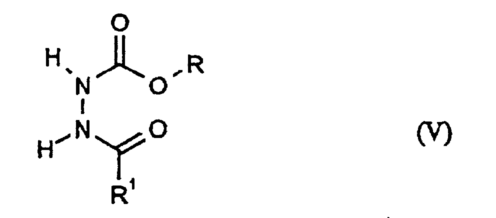 in which
R and R¹ have the abovementioned meanings,
are reacted with a sulphonyl chloride, where appropriate in the presence of an acid acceptor and where appropriate in the presence of a diluent, at temperatures of between 0°C and 100°C.

## Revendications

1. Procédé de production d'esters d'acide hydrazine carboxylique de formule générale (II) 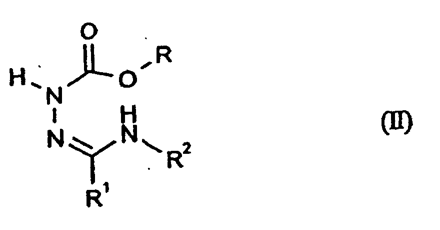 dans laquelle
R est un reste alkyle ayant 1 à 4 atomes de carbone éventuellement substitué par un halogène ou un radical alkoxy en C₁ à C₄, ou un reste phényle éventuellement substitué par un halogène ou un radical alkyle en C₁ à C₄,
R¹ est un reste halogénalkyle ayant 1 à 6 atomes de carbone et
R² représente l'hydrogène, un groupe hydroxy ou amino, un reste alkyle, alcényle, alcynyle, alkoxy, alkylamino ou dialkylamino ayant chacun jusqu'à 6 atomes de carbone dans les groupes alkyle, alcényle ou alcynyle et chacun étant éventuellement substitué par un radical cyano, halogéno, ou alkoxy en C₁ à C₄, ou un reste cycloalkyle en C₃ à C₆, (cycloalkyle en C₃ à C₆)-(alkyle en C₁ ou C₂), phényle ou phényl-(alkyle en C₁ ou C₂) dont chacun est éventuellement substitué par un radical cyano, halogéno, alkyle en C₁ à C₄, alkoxy en C₁ à C₄ ou (alkoxy en C₁ à C₄)-carbonyle,
**caractérisé en ce qu'**on fait réagir des esters d'acide hydrazine carboxylique correspondants de formule générale (III), 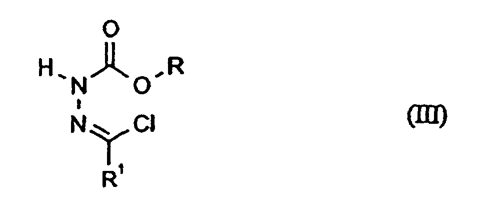 dans laquelle
R et R¹ ont les définitions indiquées ci-dessus, avec des composés aminés de formule générale (IV),
H₂N-R² (IV)
dans laquelle
R² a la définition indiquée ci-dessus,
éventuellement en présence d'un accepteur d'acide et, le cas échéant, en présence d'un diluant, à des températures comprises entre 0°C ou 100°C.

2. Esters d'acide hydrazine carboxylique de formule générale (III), 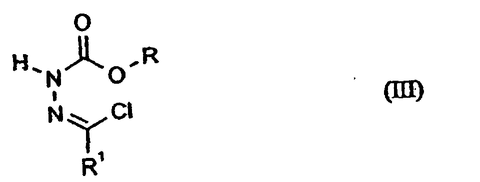 dans laquelle
R et R¹ sont tels que définis dans la revendication 1.

3. Procédé de production d'esters d'acide hydrazine carboxylique de formule générale (III), 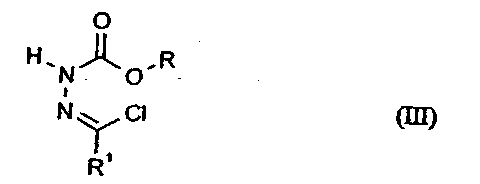 dans laquelle
R et R¹ ont des définitions indiquées dans la revendication 2,
**caractérisé en ce qu'**on fait'réagir des esters d'acide hydrazine carboxylique correspondants de formule générale (V), 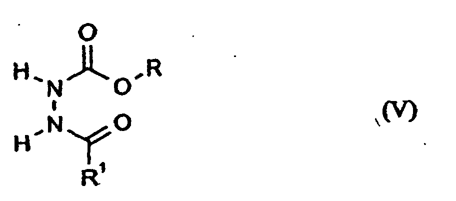 dans laquelle
R et R¹ ont les définitions indiquées ci-dessus,
avec un chlorure d'acide sulfonique, éventuellement en présence d'un accepteur d'acide et, le cas échéant, en présence d'un diluant, à des températures comprises entre 0°C et 100°C.
